(19) Europäisches Patentamt
European Patent Office
Office européen des brevets

(11) Publication number: 0 116 712
B1

(12) EUROPEAN PATENT SPECIFICATION

(45) Date of publication of patent specification: 25.03.87

(51) Int. Cl.$^4$: C 07 C 43/178, C 07 C 41/01

(21) Application number: 83112714.7

(22) Date of filing: 16.12.83

# (54) Synthesis of hindered phenols.

(30) Priority: 16.12.82 US 450207

(43) Date of publication of application:
29.08.84 Bulletin 84/35

(45) Publication of the grant of the patent:
25.03.87 Bulletin 87/13

(84) Designated Contracting States:
BE CH DE FR GB LI NL

(58) References cited:
DE-A-2 023 228
GB-A- 822 693
US-A-2 838 571
US-A-2 954 345
US-A-3 026 264
US-A-4 340 767

SOVIET INVENTIONS ILLUSTRATED, Section Ch; week C 47; January 7, 1981 DERWENT PUBLICATION LTD, London; H 06

(73) Proprietor: ETHYL CORPORATION
Ethyl Tower 451 Florida Boulevard
Baton Rouge Louisiana 70801 (US)

(72) Inventor: Mina, George Louis
Palmetto Place Apartments, Apt. D-3
Orangeburg, SC 29115 (US)

(74) Representative: Schwabe, Hans-Georg, Dipl.-Ing. et al
Patentanwälte Schwabe, Sandmair, Marx
Stuntzstrasse 16
D-8000 München 80 (DE)

EP 0 116 712 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates in general to processes for forming high molecular weight hindered phenols and intermediates therefor. This invention relates in particular to an improved catalyst/solvent system for the formation of hindered phenols.

The hindered phenols ultimately formed by the process of this invention and earlier processes have been widely used as antioxidants in food, packaging materials, stock chemicals, plastics, and the like. A process to produce such hindered phenols is set forth in Rocklin *et al*, U.S. Patent No. 3,026,264. An improvement on the Rocklin *et al* process is disclosed in my U.S. Patent No. 4,340,767.

There exists a need to reduce the processing and materials costs used in the production of antioxidants such as 1,3,5-trimethyl-2,4,6-tris(3,5-di-*tert*-butyl-4-hydroxybenzyl)-benzene. The prior art processes have used, for example, 3,5-dialkyl-4-hydroxybenzyl alcohol (also called 2,6-dialkyl-α-hydroxy-p-cresol) as an intermediate to produce the high molecular weight hindered phenols. The alcohol intermediates must be isolated from their reaction mass as a solid by means of centrifugation which is quite expensive. Also, adequate washing of the solid filter cake has proven difficult and some of the wash solvents may be carried forward into the product causing various problems.

The 3,5-di-*tert*-butyl-4-hydroxybenzyl alcohol is often used as an intermediate. However, this intermediate is insoluble in preferred solvents such as methylene chloride. Thus strict stoichiometry controls are required and undesirable side reactions with sulfuric acid catalyst may occur. Finally, the use of an alcohol intermediate produces significant amounts of "heel" and certain bisphenols which, while usable, tie the subject process to other processes and markets.

The substitution of alkyl ethers of intermediate alcohols has been suggested to produce high molecular weight hindered phenols. However, the ethers are not always available and their production has not been cost effective.

The present invention is directed to the reduction of costs in the production of high molecular weight hindered phenols, commonly used as antioxidants. The present invention is a process for the production of aromatic ethers of structure I:

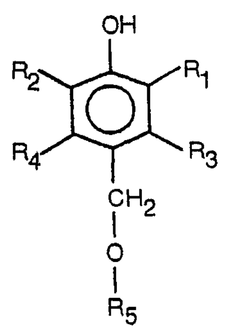

(I)

comprising reacting in the presence of a Mannich base catalyst: formaldehyde; a stoichiometric excess of an alcohol of structure II:

$$R_5\text{—OH} \qquad \text{(II)}$$

where $R_5$ is alkyl or cycloalkyl; and a substituted phenol of structure III:

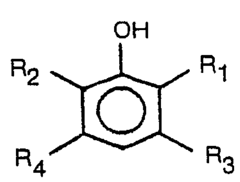

(III)

where $R_1$ and $R_2$ are the same or different and are selected from alkyls, cycloalkyls, aryls, aralkyls, and alkaryls; and $R_3$ and $R_4$ are the same or different and are selected from H, alkyls, cycloalkyls, aryls, alkaryls, and aralkyls.

The present invention is also a process for the production of high molecular weight hindered phenols, said process comprising the steps of:

(a) reacting in the presence of a Mannich base catalyst:

(i) formaldehyde,

(ii) a stoichiometric excess of an alcohol of structure II:

$R_5$—OH (II)

where $R_5$ is alkyl or cycloalkyl, and

(iii) a substituted phenol of structure III:

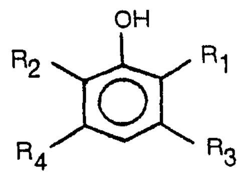

(III)

where $R_1$ and $R_2$ are the same or different and are selected from alkyls, cycloalkyls, aryls, aralkyls, and alkaryls; and $R_3$ and $R_4$ are the same or different and are selected from H, alkyls, cycloalkyls, aryls, aralkyls, and alkaryls so as to form an aromatic ether of structure I:

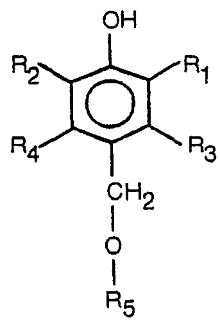

(I)

(b) removing the excess unreacted alcohol;

(c) adding a hydrocarbon or halogenated hydrocarbon solvent; and

(d) reacting the aromatic ether of structure (I) with benzene or an alkylated benzene.

The present invention is also a process for the production of 1,3,5-trimethyl-2,4,6-tris(3,5-di-*tert*-butyl-4-hydroxybenzyl)benzene with conservation of catalyst materials, elimination of centrifugation of a chemical intermediate, and minimization of by-product bisphenol intermediate, said process comprising the steps of:

(a) reacting 2,6-di-*tert*-butylphenol, formaldehyde, and a stoichiometric excess of methanol in the presence of a catalytic portion of 2,6-di-*tert*-butyl-4-dialkylaminomethylphenol so as to form the intermediate 2,6-di-*tert*-butyl-α-methoxy-*p*-cresol, said catalytic portion being formed from a dialkyl secondary amine, whereby some of the secondary amine remains in the unreacted excess methanol and formation of 4,4′-methylenebis(2,6-di-*tert*-butylphenol) is minimized;

(b) recovering the unreacted excess methanol containing some of the secondary amine and recycling said methanol to another intermediate formation reaction thereby conserving catalyst;

(c) dissolving the intermediate 2,6-di-*tert*-butyl-α-methoxy-*p*-cresol in methylene chloride thereby eliminating the need for centrifugation;

(d) reacting the dissolved intermediate with mesitylene in the presence of a sulfuric acid or Friedel-Crafts catalyst; and

(3) recovering the 1,3,5-trimethyl-2,4,6-tris(3,5-di-*tert*-butyl-4-hydroxybenzyl)benzene product.

The present invention has been demonstrated as a clean process for the production of certain ethers of hindered *para* cresols as intermediate compounds. The invention is also a process to produce high molecular weight hindered phenols formed from the intermediate aromatic ethers and benzene compound to be combined therewith.

The aromatic ether compounds of the invention are of the general structure I:

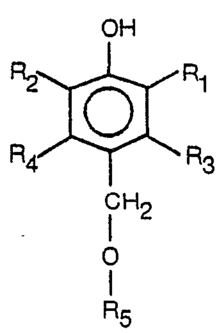

(I)

where $R_1$ and $R_2$ are the same or different and are selected from straight chain or (preferably) branched alkyls, cycloalkyls, aryls, aralkyls, and alkaryls; $R_3$ and $R_4$ are the same or different and are selected from H (preferably), alkyls, cycloalkyls, aryls, alkaryls, and aralkyls; and $R_5$ is alkyl (preferably of one to six carbon atoms) or cycloalkyl of 5—12 carbon atoms.

The alcohols of the invention are of the general structure II:

$$R_5\text{—OH} \qquad \text{(II)}$$

where $R_5$ is as defined above. These alcohols include methanol, isopropanol, ethanol, *sec*-butanol, *n*-hexanol, cyclopentanol, isoamyl alcohol, *n*-propanol, *n*-butanol, and others. Preferred alcohols are the lower alkyl alcohols such as methanol, isopropanol, *sec*-butanol, and *n*-hexanol. More preferred are the unbranched lower alkyl alcohols. These include methanol, *n*-butanol, ethanol, *n*-hexanol, and the like. Most preferred are the $C_1$—$C_4$ straight chain alkyl alcohols. Most especially preferred is methanol since methanol reacts quickly and completely in the inventive process, is readily available, and carries catalyst over into the recycled excess methanol when used as a solvent/reactant.

The cycloalkyls and branched alkyl groups are preferred substituents for $R_1$ and $R_2$. Branched alkyls of one to six carbon atoms and cycloalkyls of five and six carbon atoms are more preferred and the *tert*-butyl group is the most preferred substituent for $R_1$ and $R_2$ because of its strong hindrance effect for phenols, especially where an antioxidant use is proposed.

The substituted phenols of the invention have the general structure III:

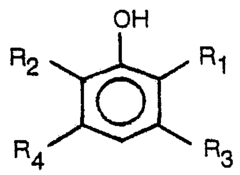

(III)

where $R_1$, $R_2$, $R_3$, and $R_4$ are as defined above. Examples of the phenols suitable to make the aromatic ether of the invention include:

2,6-di-*tert*-butylphenol;
2,6-diisopropylphenol;
2,6-*sec*-butylphenol;
2-methyl-6-*tert*-butylphenol;
2,6-diethylphenol;
2,6-dicyclopentylphenol;
2,6-dicyclohexylphenol;
2-cyclopentyl-6-*tert*-butylphenol;
2,6-diisopropyl-3-methylphenol;
2,6-di-*tert*-butyl-3,5-dimethylphenol;
2,3,6-triisopropylphenol;
2,3,6-triethylphenol;

and the like. More preferred are the 2,6-di-lower alkylphenols and 2,6-di-lower cycloalkylphenols, otherwise unsubstituted, i.e., where $R_3$ and $R_4$ are both H. These include:

2,6-di-*tert*-butylphenol;
2,6-dimethylphenol;
2,6-dicyclopentylphenol;
2,6-diisopropylphenol;
2-methyl-6-*tert*-butylphenol;
2-ethyl-6-*sec*-butylphenol;

and the like.

Most preferred are those 2,6-di-lower-alkylphenols otherwise unsubstituted where the lower alkyl substituents are identical such as 2,6-diisopropylphenol, 2,6-di-*sec*-butylphenol, and 2,6-di-*tert*-butylphenol. Most highly preferred is the compound 2,6-di-*tert*-butylphenol.

The intermediate ethers of the invention include but are not limited to:

2,6-di-*tert*-butyl-α-methoxy-*p*-cresol;
2,6-di-*tert*-butyl-α-ethoxy-*p*-cresol;
2,6-di-*tert*-butyl-3,5-dimethyl-α-methoxy-*p*-cresol;
2,6-dicyclopentyl-α-methoxy-*p*-cresol;
2,6-di-*tert*-amyl-α-butoxy-*p*-cresol;
2,6-di-*sec*-butyl-α-cyclohexoxy-*p*-cresol;
2,6-dicyclohexyl-α-methoxy-*p*-cresol;
2,6-dimethyl-α-propoxy-*p*-cresol;
2-methyl-6-*tert*-butyl-α-methoxy-*p*-cresol;

2-ethyl-6-octadecyl-α-ethoxy-*p*-cresol;
2-methyl-6-cyclooctyl-α-methoxy-*p*-cresol;
2-α,α-dimethylbenzyl-6-methyl-α-methoxy-*p*-cresol;
2-methyl-6-isopropyl-α-cyclopentoxy-*p*-cresol;
and others.

Most secondary amines are acceptable for the process. The preferred amines have the structure IV:

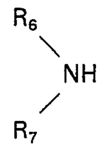

(IV)

wherein $R_6$ and $R_7$ are separately the same or different and are selected from alkyl, cycloalkyl, alkanol, cycloalkanol, aromatic, heterocyclic, or together with the nitrogen to which they are attached form a ring.

Representative examples of these amines are dimethylamine, diethylamine, methylethylamine, diisoamylamine, dibenzylamine, methylisobutylamine, diisobutylamine, dicyclohexylamine, methylcyclohexylamine, ethylcyclopentylamine, methylcyclooctylamine, diethanolamine, methylethanolamine, methyl(2-hydroxybutyl)amine, methyl(2-hydroxycyclohexyl)amine, ethyl(4-hydroxycyclohexyl)amine, N-methylaniline, methyl-*o*-tolylamine, dibenzylamine, methylbenzylamine, methyl(α-methylbenzyl)amine, N-(N-[3-aminopropyl]morpholine)-N-methylamine, piperidine, piperazine, morpholine, and the like.

The dialkylamines, dicycloalkylamines, and alkanolamines are more preferred since they are readily available, cheap, and selectively form Mannich base compounds. While long chain alkylamines are usable, their use may require selection of an expensive solvent/reaction to form the ether intermediate. Included among the more preferred amines are dimethylamine, diethylamine, dipropylamine, di-*n*-butylamine, diisoamylamine, methylethylamine, diisopropylamine, didodecylamine, methylisopropylamine, and the like. Still more preferably $R_6$ and $R_7$ are lower alkyl groups containing one to four carbon atoms such as dimethylamine, diethylamine, methylethylamine, diisopropylamine, methylisobutylamine, and the like. The most preferred dialkylamines are dimethylamine and diethylamine, especially dimethylamine.

Preferred alkanolamines are those in which the alkanol groups contain about two to four carbon atoms such as diethanolamine, methylethanolamine, di-(2-hydroxypropyl-)amine, di-(2-hydroxybutyl)amine, ethylethanolamine, isobutylethanolamine, and the like. The most preferred alkanol amines are the dialkanolamines especially diethanolamine. Also suitable is methyl-N,N'-diethylethylenediamine. Other usable secondary amines include piperidine, 1,2,3,4-tetrahydroisoquinoline, 6-methoxy-1,2,3,4-tetrahydroisoquinoline, morpholine, piperazine, ω-methylaminopropiophenone, β-acetylethylbenzylamine, benzyl-(2-cyclohexanonylmethyl)amine, and 3,4-methylenedioxybenzyl-(2-cyclohexanonylmethyl)amine.

Dimethylamine is the most preferred of all secondary organic amines because it readily forms catalyst in the preferred solvent, methanol. Mixtures of secondary amines may be used.

The invention uses a Mannich base catalyst to produce the alkyl ethers of *para*-cresols. The Mannich base catalyst is readily formed by the addition of a secondary organic amine to a combination of formaldehyde and a hindered phenol such as 2,6-di-*tert*-butylphenol or 2,6-di-cyclopentylphenol.

The catalyst of the invention may be formed *in situ* with formaldehyde and the substituted phenol by addition of a suitable secondary amine to form a structure V:

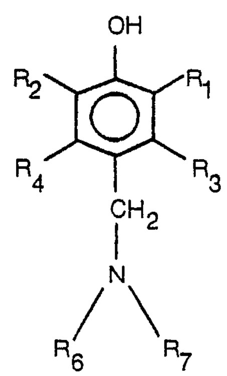

(V)

where $R_1$, $R_2$, $R_3$, $R_4$, $R_6$, and $R_7$ are as defined above. Such Mannich base compounds can also be used in the process of the invention by their simple addition to the (substituted) benzene and a compound of structure (I), with appropriate catalyst at suitable reaction conditions.

Examples of Mannich base compounds of structure (V) are:
2,6-di-*tert*-butyl-dimethylaminomethylphenol;
2,6-diisopropyl-dimethylaminomethylphenol;
2,6-di-*tert*-butyl-diethylaminomethylphenol;

2-methyl-6-*tert*-butyl-dimethylaminomethylphenol;
2,6-diethyl-diethanolaminomethylphenol;
and the like.

The high molecular weight hindered phenols according to the invention include those formed from the above ethers and a benzene or alkylated benzene. The methyl substituted benzenes such as toluene, xylene isomers, trimethylbenzene isomers, and durene are preferred. Mesitylene(1,3,5-trimethylbenzene) is most preferred since it may be used to form the valuable antioxidant 1,3,5-trimethyl-2,4,6-tris(3,5-di-*tert*-butyl-4-hydroxybenzyl)benzene and similar homologs. Other suitable compounds include 1,3,5-triethylbenzene, 1,4-diisopropylbenzene, 1,3-dimethyl-5-ethylbenzene, 1,2,4,5-tetramethylbenzene, 1,2-dimethyl-4,5-diethylbenzene, 1-*n*-butyl-3-ethyl-5-methylbenzene, and the like.

Preferably, a small amount of dimethylamine is added to a 2,6-dialkylphenol, formaldehyde (flake or *para*-formaldehyde), and methanol (sufficiently in excess to act as solvent) *in situ*. Heating promotes catalyst formation, thus the amine may merely be added at the start of the reaction sequence so as to form the catalyst for reaction. The preferred catalysts are the 2,6-dialkyl-α-dimethylamino-*p*-cresols. The compound 2,6-di-*tert*-butyl-α-dimethylamino-*p*-cresol is most preferred.

When a 2,6-dialkylphenol is used as the limiting reactant, dimethylamine remains dissolved in the excess methanol or other reactant/solvent and is thus carried over for reuse in subsequent reactions.

Both sulfuric acid and Friedel-Crafts catalysts are suitable according to the invention for formation of high molecular weight hindered phenolic compounds. Sulfuric acid is preferred but suitable Friedel-Crafts catalysts include $AlCl_3$, $AlBr_3$, $FeCl_3$, and the like.

Suitable solvents for reaction of a benzene or alkylbenzene with the alkyl ethers of the invention include various hydrocarbons and, preferably, halogenated hydrocarbons. These include the paraffins, especially $C_5$—$C_{10}$ compounds such as pentane, isopentane, hexane, cyclohexane, heptane, octane, isooctane, and decane as well as mixtures of these. Also included are alkylene halides such as 1,1-dichloropropane, 1,2-dichloropropane, 2,2-dichloropropane, 1,1-dichloroethane, 1,2-dichloroethane, 1,1,1-trichloroethane, *n*-butylchloride, *sec*-butylchloride, isobutylchloride, chloroform, carbon tetrachloride, and the like. Methylene chloride is the especially preferred solvent for production of the high molecular weight hindered phenols.

The amount of solvent may vary so long as dissolution of the ingredients is achieved to the extent required for reaction. A useful range is 50—500 parts solvent per 100 parts reactant phenol. A preferred range is from 100—200 parts solvent per 100 parts reactant.

The compounds made by this invention have proven antioxidant activity and are, therefore, very useful.

The present invention is especially advantageous over prior art processes not only because of its one pot procedure but also because it minimizes the production of bisphenol coproducts, notably 4,4'-methylenebis(2,6-di-*tert*-butylphenol).

Earlier attempts to prepare the chemical intermediate of the invention used a strong base catalyst such as potassium hydroxide (KOH).

In the DE-OS 2023228 a process for preparation of the relevant compound, α-alkoxy-3,5-di-tert-butylcresol, is described comprising the reacting of 2,6 di-tert-butylphenol with formaldehyde and alcohols, said reaction is catalysed by a base, like KOH. In a further process the catalyst used is pyridine or triethylamine.

The GB—PS 832693 relating to substituted phenols useful as age-resisters, describes a process wherein said substituted phenols can be produced from phenols which are correspondingly substituted in the 2-position and 6-position by reaction of the substituted phenols with an aliphatic or aromatic aldehyde, such as for example formaldehyde, in alcoholic solution in the presence of compounds having an alcaline reaction.

The US—PS 2954345, relating to 3,5-dialkyl-4-hydroxybenzylether useful as anti-oxidants describes a method for preparation of said compounds comprising the reaction of a 2,6-dialkylphenol, formaldehyde and a monohydric alcohol in the presence of a catalytic quantity of a metallic hydroxide condensation catalyst, the metallic hydroxide being of a metal selected from the group consisting of alkali and alkaline earth metals.

The US—PS 2838571, relating to 3,5-dialcyl-4-hydroxybenzylethers, describes a method for preparing said compounds by reacting a 2,6-dialkylphenol, formaldehyde and a mono-hydric alcohol in the presence of a catalytic quantity of a metallic hydroxide condensation catalyst, said metallic hydroxide being of a metal selected from the group consisting of alkali and alkaline earth metals.

This resulted in the conversion of 25 percent or more of the starting hindered phenol to a bisphenol. While the bisphenol is a usable compound, it is not nearly as valuable as the chemical intermediate of the invention. Furthermore, such prior art processes unnecessarily tied the production of high molecular weight hindered phenols to a relatively lower weight phenolic. When the inventive process is operated with 2,6-di-*tert*-butylphenol, the conversion to 4,4'-methylenebis(2,6-di-*tert*-butylphenol) is as low as five percent or less with nearly complete conversion to the desired chemical intermediate.

According to the invention, the formaldehyde, hindered phenol, excess lower alcohol, and small amount of catalyst are heated to an elevated temperature in a sealed reaction chamber thereby raising the pressure. A suitable reaction temperature range is 50°—200°C., preferably 60°—130°C. A suitable range of reaction pressure is 0—69 bar, preferably 0,35—6,9 bar. More preferably the reaction is carried out with

methanol at about 60°—100°C. and 0,35—1,73 bar. Such conditions tend to limit the formation of compounds of the type 4,4'-methylenebis-(2,6-dialkylphenol).

The procedure of the preferred embodiment permits a one pot process whereby excess solvent from formation of the alkyl ether of the phenol reactant is stripped off, the appropriate solvent for reaction with, e.g., mesitylene is added, and the high molecular weight hindered phenol is formed *in situ*. This obviates the need for severe mechanical separation of the intermediate such as by centrifuge.

The preparation of phenolic compounds by the inventive process may be carried out in several fashions and the ingredients may be combined in any order prior to reaction. Preferably, the acid catalyst is added last. Multiple additions of catalyst may be made.

The ratio of reactants for preparation of the phenolic may be combined over a broad range, but excess solvent is preferred for better reaction mixing.

A slight excess of the structure (I) ether over stoichiometric is preferable since excess benzene or alkylated benzene may otherwise lead to an undesirable mixture of monosubstituted and polysubstituted benzenes.

The preparation of phenolic may be carried out over a broad range of temperatures but −60° to 140°C. is a usable range. From −10°C. to 30°C. is a preferred range and from 0° to 10°C. is more preferred.

Although superatmospheric and subatmospheric pressures may be used, they are unnecessary or uneconomical where a sufficiently active catalyst is used.

## Example

*Preparation of Ether*

Toa 500 ml., 4 neck, round bottom flask equipped with mechanical stirrer, nitrogen inlet, reflux condenser, and side arm dropping funnel was charged the following:

220 ml. methanol;
24.0 grams paraformaldehyde;
3.0 grams 40% dimethylamine in water.

The mixture was heated to reflux (about 68°C.) and a solution of 103.2 grams 2,6-di-*tert*-butylphenol in 50 ml. methanol was added dropwise over a period of three hours under nitrogen flush and at reflux. The reaction mixture was transferred to a one-liter Parr pressure reactor equipped with dual pitch blade impellers. The reactor was heated to and maintained at 80°C. with stirring for four hours, whereupon the pressure in the reactor reached about 1,0—1,38 bar. The reaction mixture was cooled to about 65°C. and transferred to a one-liter Morton, creased, three neck, round bottom flask equipped with a mechanical stirrer having a three-inch, half moon impeller, a thermometer, and a side dropping funnel distillation head. The reaction mixture was heated in the flask in a 100°—120°C. oil bath under nitrogen flush with agitation to strip off solvent. When the reaction mass temperature reached 95°C. the oil bath was removed and an ice bath applied. Analysis confirmed formation of 2,6-di-*tert*-butyl-α-methoxy-*p*-cresol.

*Preparation of high molecular-weight hindered phenol*

The cooled Morton flask was set up for reflux and the following were added to the reaction mixture:

200 ml. methylene chloride;
14.8 grams mesitylene; and
1.5 ml. acetic acid.

The flask was cooled to 5°C. in an ice bath and stirred at 150 rpm. While maintaining the reaction mixture at 3°—7°C., about 25 ml. 84% aqueous $H_2SO_4$ was added dropwise over two hours. The reaction mass was transferred to a separatory funnel and allowed to settle for about 15 minutes. The lower, acid phase was cut off and the remainder of the reaction mass was returned to the one-liter Morton flask. A second portion of 1.5 ml. acetic acid was charged and a second 25 ml. portion of 84% $H_2SO_4$ was added dropwise over two hours as above. The same procedure was followed to separate the acid phase. Thereupon the remaining reaction mass was charged to a one-liter resin flask equipped with four neck top, bottom stopcock, mechanical agitator, thermometer, nitrogen flush, and distillation/reflux head. To the reaction mixture is then added 360 ml. deionized water and 100 grams soda ash. The reaction mixture was heated by means of the steam jacket to strip off methylene chloride until no more comes off overhead with a pot temperature of at least 85°C. The flask was set up for reflux and 800 ml. heptane added. The reaction mass was heated to reflux and all solids dissolved whereupon the mass was allowed to settle for about five minutes before cutting off the lower, aqueous phase. The reaction mass was washed twice with two 200 ml. portions of deionized water and a pH of about 5—7 was determined. The hot heptane solution was transferred to a one liter, three neck, round bottom flask equipped with mechanical stirrer, thermometer, and nitrogen flask. The solution was cooled slowly to 5°C. and the resultant slurry was filtered on a buchner funnel. The product was washed with 140 ml. heptane and dried to provide 83.25 grams 1,3,5-trimethyl-2,4,6-tris(3,5-di-*tert*-butyl-4-hydroxybenzyl)benzene of over 99 percent purity by high pressure liquid chromatography analysis.

The above example demonstrates formation of the catalyst *in situ* but of course the catalyst could be separately added.

**Claims**

1. A process for the production of aromatic ethers of structure I:

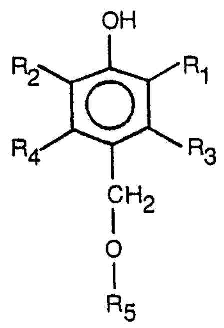

(I)

comprising reacting formaldehyde, a stoichiometric excess of an alcohol of structure II:

$$R_5—OH \quad (II)$$

where $R_5$ is alkyl or cycloalkyl; and a substituted phenol of structure III:

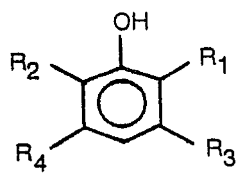

(III)

where $R_1$ and $R_2$ are the same or different and are selected from alkyls, cycloalkyls, aryls, aralkyls, and alkaryls; and $R_3$ and $R_4$ are the same or different and are selected from H, alkyls, cycloalkyls, aryls, alkaryls, and aralkyls, characterised in that said reaction is carried out in the presence of a Mannich base catalyst.

2. The process as claimed in Claim 1 in which said alcohol is methanol.

3. The process as claimed in Claim 1 in which said substituted phenol of structure (III) has $R_3$ and $R_4$ both H and $R_1$ and $R_2$ are alkyl or cycloalkyl.

4. The process as claimed in Claim 3 in which $R_1$ and $R_2$ are *tert*-butyls.

5. The process as claimed in Claim 1 in which said Mannich base is formed *in situ* from a portion of said substituted phenol, formaldehyde and a catalyst-forming portion of a secondary amine.

6. The process as claimed in Claim 5 in which said secondary amine is of structure (IV):

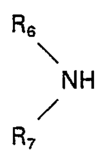

(IV)

wherein $R_6$ and $R_7$ are separately the same or different and are selected from alkyl, cycloalkyl, alkanol, cycloalkanol, aromatic, heterocyclic, or together with the nitrogen to which they are attached form a ring.

7. The process as claimed in Claim 5 in which said secondary amine is diethylamine or dimethylamine.

8. The process as claimed in Claim 1 in which said formaldehyde is an aqueous solution, flake, or paraformaldehyde.

9. The process as claimed in Claim 1 in which said Mannich base is of structure V:

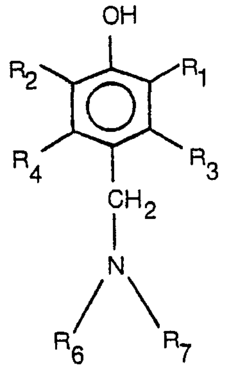

(V)

wherein $R_6$ and $R_7$ are separately the same or different and are selected from alkyl, cycloalkyl, alkanol, cycloalkanol, aromatic, heterocyclic, or together with the nitrogen to which they are attached form a ring.

10. The process as claimed in Claim 1 in which said Mannich base catalyst is 2,6-dialkyl-4-dimethylaminomethylphenol.

11. The process as claimed in Claim 1 in which said reaction is carried out at a temperature of 50°—200°C.

12. The process as claimed in Claim 1 carried out at a pressure of 1,35 to 8 bar and at an elevated temperature.

13. A process for the production of high molecular weight hindered phenols, said process comprising the steps of:

(a) reacting in the presence of a Mannich base catalyst formaldehyde, alcohol and a substituted phenol according to claim 1 so as to form an aromatic ether of structure I:

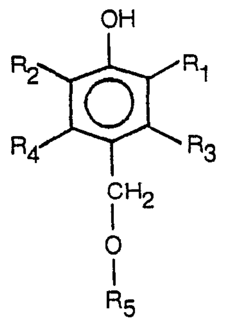

(I)

(b) removing the excess unreacted alcohol;

(c) adding a hydrocarbon or halogenated hydrocarbon solvent; and

(d) reacting the aromatic ether of structure (I) with benzene or an alkylated benzene.

14. A process for the production of 1,3,5-trimethyl-2,4,6-tris(3,5-di-tert.-butyl-4-hydroxybenzyl)benzene said process comprising the steps of:

(a) reacting 2,6-di-tert-butylphenol, formaldehyde, and a stoichiometric excess of methanol in the presence of a catalytic portion of 2,6-di-tert-butyl-4-dialkylaminomethylphenol according to any of the preceding claims 1—12 so as to form the intermediate 2,6-di-tert-butyl-α-methoxy-p-cresol, said catalytic portion being formed from a dialkyl secondary amine, whereby some of the secondary amine remains in the unreacted excess methanol;

(b) recovering the unreacted excess methanol containing some of the secondary amine and recycling said methanol to another intermediate formation reaction thereby conserving catalyst;

(c) dissolving the intermediate 2,6-di-tert-butyl-α-methoxy-p-cresol in methylene chloride thereby eliminating the need for centrifugation;

(d) reacting the dissolved intermediate with mesitylene in the presence of a sulfuric acid or Friedel-Crafts catalyst; and

(e) recovering the 1,3,5-trimethyl-2,4,6-tris(3,5-di-tert-butyl-4-hydroxybenzyl)benzene product.

**Patentansprüche**

1. Verfahren zur Herstellung aromatischer Ether der Formel (I):

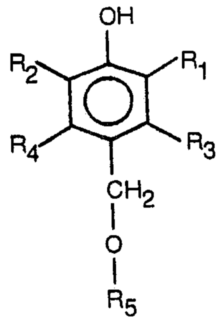

(I)

durch Umsetzung von Formaldehyd, eines stöchiometrischen Überschusses eines Alkohols der Formel (II)

$R_5$—OH (II)

worin $R_5$ eine Alkylgruppe oder Cycloalkylgruppe bedeutet und eines substituierten Phenols der Formel (III)

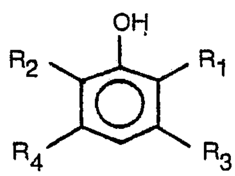

(III)

worin $R_1$ und $R_2$ gleich oder verschieden sind und aus Alkyl, Cycloalkyl, Aryl, Aralkyl und Alkaryl ausgewählt sind, $R_3$ und $R_4$ gleich oder verschieden sind und aus Wasserstoff, Alkyl, Cycloalkyl, Aryl, Alkaryl und Aralkyl ausgewählt sind, dadurch gekennzeichnet, daß die Umsetzung in Anwesenheit eines Mannich-Base-Katalysators durchgeführt wird.

2. Verfahren nach Anspruch 1, bei dem der Alkohol Methanol ist.

3. Verfahren nach Anspruch 1, bei dem im substituierten Phenol der Formel (III) $R_3$ und $R_4$ Wasserstoff und $R_1$ und $R_2$ Alkyl oder Cycloalkyl bedeuten.

4. Verfahren nach Anspruch 3, bei dem $R_1$ und $R_2$ tert-Butylreste sind.

5. Verfahren nach Anspruch 1, bei dem die Mannich-Base in situ aus einem Teil des substituierten Phenols, Formaldehyd und einem katalysator-bildenden Teil eines sekundären Amins erzeugt wird.

6. Verfahren nach Anspruch 5, bei dem das sekundäre Amin die Formel (IV) aufweist:

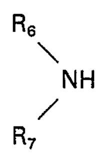

(IV)

worin $R_6$ und $R_7$ unabhängig voneinander gleich oder verschieden sind und aus Alkyl, Cycloalkyl, Alkanol, Cycloalkanol, Aromaten, heterocyclischen Resten ausgewählt sind, oder zusammen mit dem Stickstoffatom, an das sie gebunden sind, einen Ring bilden.

7. Verfahren nach Anspruch 5, bei dem das sekundäre Amin Diethylamin oder Dimethylamin ist.

8. Verfahren nach Anspruch 1, bei dem der Formaldehyd eine wäßrige Lösung, Flocken- oder Paraformaldehyd ist.

9. Verfahren nach Anspruch 1, bei dem die Mannich-Base die Formel (V) aufweist:

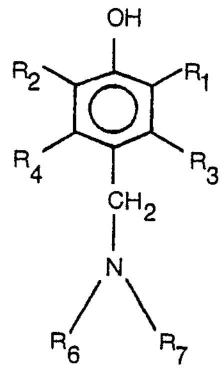

(V)

worin $R_6$ und $R_7$ unabhängig voneinander gleich oder verschieden sind und aus Alkyl, Cycloalkyl, Alkanol, Cycloalkanol, aromatischen Resten, heterocyclischen Resten ausgewählt sind oder zusammen mit dem Stickstoffatom, an das sie gebunden sind, einen Ring bilden.

10. Verfahren nach Anspruch 1, bei dem der Mannich-Base-Katalysator 2,6-Dialkyl-4-dimethylaminomethylphenol ist.

11. Verfahren nach Anspruch 1, bei dem die Reaktion bei einer Temperatur von 50 bis 200°C durchgeführt wird.

12. Verfahren nach Anspruch 1, dadurch gekennzeichnet, daß das Verfahren bei einem Druck von 1,35 bis 8 bar und bei einer erhöhten Temperatur druchgeführt wird.

13. Verfahren zur Herstellung sterisch gehinderten Phenolen mit hohem Molekulargewicht durch

(a) Umsetzung von Formaldehyd, Alkohol und eines substituierten Phenols gemäß Anspruch 1 in Gegenwart eines Mannich-Base-Katalysators zur Bildung eines aromatischen Ethers der Formel (I)

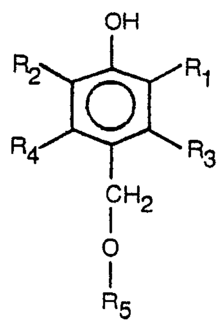

(I)

(b) Entfernung des Überschusses des nicht-reagierten Alkohols;

(c) Zugabe eines Kohlenwasserstoffs oder eines halogenierten Kohlenwasserstofflösungsmittels; und

(d) Umsetzung des aromatischen Ethers der Formel (I) mit Benzol oder einem alkylierten Benzol.

14. Verfahren zur Herstellung von 1,3,5-Trimethyl-2,4,6-tris(3,5-di-tert-butyl-4-hydroxybenzyl)-benzol durch

(a) Umsetzung von 2,6-di-tert-Butylphenol, Formaldehyd und einem Stöchiometrischen Überschuß von Methanol in Anwesenheit einer katalytischen Menge von 2,6-di-tert-Butyl-4-dialkylaminomethylphenol gemäß einem der Ansprüche 1 bis 12, zur Bildung des Zwischenprodukts 2,6-di-tert-Butyl-α-methoxy-p-kresol, wobei der katalytische Anteil aus einem Dialkyl-sekundären Amin gebildet wird, wodurch ein Teil des sekundären Amins im nicht-reagierten überschüssigen Methanol verbleibt;

(b) Rückgewinnung des nicht-reagierten überschüssigen Methanols, das einen Teil des sekundären Amins enthält, und Zurückführung des Methanols zu einer weiteren Zwischenproduktbildung, um dadurch den Katalysator zu erhalten;

(c) Auflösung des Zwischenprodukts 2,6-di-tert-Butyl-α-methoxy-p-kresol in Methylenchlorid, um die erforderliche Zentrifugation zu umgehen;

(d) Umsetzung des gelösten Zwischenproduktes mit Mesitylen in Anwesenheit einer Schwefelsäure oder eines Friedel-Craft-Katalysators; und

(e) Rückgewinnung von 1,3,5-Trimethyl-2,4,6-tris(3,5-di-tert-butyl-4-hydroxybenzyl)-benzol.

**Revendications**

1. Procédé de production d'éthers aromatiques de formule I:

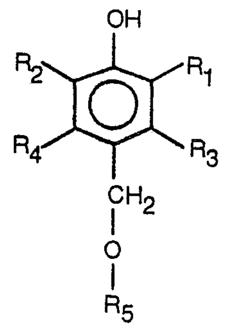

(I)

comprenant la réaction de formaldéhyde, d'un excès stoechiométrique d'un alcool de formule II:

$R_5$—OH (II)

dans laquelle $R_5$ est un groupe alkyle ou cycloalkyle; et d'un phénol substitué de formule III:

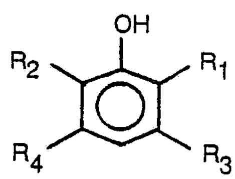

(III)

dans laquelle $R_1$ et $R_2$ sont identiques ou différents et sont choisis entre des groupes alkyle, cycloalkyle, aryle, aralkyle et alkaryle; et $R_3$ et $R_4$ sont identiques ou différents et sont choisis entre l'hydrogène et des groupes alkyle, cycloalkyle, aryle, alkaryle et aralkyle, caractérisé en ce que ladite réaction est conduite en présence d'un catalyseur du type d'une base de Mannich.

2. Procédé suivant la revendication 1, dans lequel ledit alcool est le méthanol.

3. Procédé suivant la revendication 1, dans lequel $R_3$ et $R_4$ sont tous deux de l'hydrogène et $R_1$ et $R_2$ sont des groupes alkyle ou cycloalkyle dans le phénol substitué de formule (III).

4. Procédé suivant la revendication 3, dans lequel $R_1$ et $R_2$ sont des groupes tertio-butyle.

5. Procédé suivant la revendication 1, dans lequel ladite base de Mannich est formée in situ à partir d'une portion dudit phénol substitué, de formaldéhyde et d'une portion formant catalyseur d'une amine secondaire.

6. Procédé suivant la revendication 5, dans lequel ladite amine secondaire répond à la formule (IV):

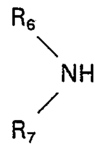

(IV)

dans laquelle $R_6$ et $R_7$, considérés séparément, sont identiques ou différents et sont choisis entre des groupes alkyle, cycloalkyle, alcanol, cycloalcanol, aromatiques, hétérocycliques, ou forment un noyau conjointement avec l'atome d'azote auquel ils sont liés.

7. Procédé suivant la revendication 5, dans lequel ladite amine secondaire est la diéthylamine ou la diméthylamine.

8. Procédé suivant la revendication 1, dans lequel le formaldéhyde est en solution aqueuse, en paillettes ou sous la forme de para-formaldéhyde.

9. Procédé suivant la revendication 1, dans lequel ladite base de Mannich répond à la formule V:

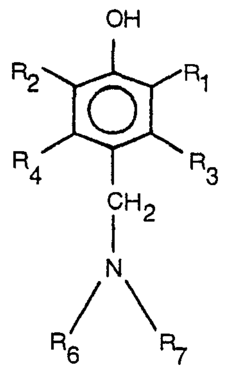

(V)

dans laquelle $R_6$ et $R_7$, considérés séparément, sont identiques ou différents et sont choisis entre des groupes alkyle, cycloalkyle, alcanol, cycloalcanol, aromatiques, hétérocycliques ou forment un noyau conjointement avec l'atome d'azote auquel ils sont liés.

10. Procédé suivant la revendication 1, dans lequel le catalyseur du type d'un base de Mannich est un 2,6-dialkyl-4-diméthylaminométhylphénol.

11. Procédé suivant la revendication 1, dans lequel ladite réaction est conduite à une température de 50 à 200°C.

12. Procédé suivant la revendication 1, mis en oeuvre à une pression de 1,35 à 8 bars et à une température élevée.

13. Procédé de production de phénols à encombrement stérique de haut poids moléculaire, procédé qui comprend les étapes consistant:

(a) à faire réagir en présence d'un catalyseur du type d'une base de Mannich, du formaldéhyde, un alcool et un phénol substitué suivant la revendication 1 de manière à former un éther aromatique de formule I:

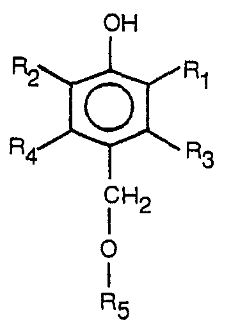

(I)

(b) à chasser l'alcool en excès n'ayant pas réagi;

(c) à ajouter comme solvant un hydrocarbure ou un hydrocarbure halogèné; et

(d) à faire réagir l'éther aromatique de formule (I) avec du benzène ou un benzène alkylé.

14. Procédé de production de 1,3,5-triméthyl-2,4,6-tris-(3,5-di-tertio-butyl-4-hydroxybenzyl)benzène, caractérisé en ce qu'il comprend les étapes consistant:

(a) à faire réagir du 2,6-di-tertio-butylphénol, du formaldéhyde et un excès stoechiométrique de méthanol en présence d'une portion catalytique de 2,6-di-tertio-butyl-4-dialkylaminométhylphénol conformément à l'une quelconque des revendications 1 à 12 précédentes de manière à former le 2,6-di-tertio-butyl-α-méthoxy-*p*-crésol intermédiaire, ladite portion catalytique étant formée à partir d'une dialkylamine secondaire, en sorte qu'une certaine quantité de l'amine secondaire reste dans le méthanol en excès n'ayant pas réagi;

(b) à récupérer le méthanol en excès n'ayant pas réagi contenant une certaine quantité de l'amine secondaire et à recycler ledit méthanol dans une autre réaction de formation de composé intermédiaire de manière à conserver le catalyseur;

(c) à dissoudre le 2,6-di-tertio-butyl-α-méthoxy-p-crésol intermédiaire dans du chlorure de méthylène de manière à eliminer la nécessité d'une centrifugation;

(d) à faire réagir le composé intermédiaire dissous avec du mésitylène en présence d'un catalyseur consistant en acide sulfurique ou en un catalyseur de Friedel-Crafts; et

(e) à recueillir comme produit le 1,3,5-triméthyl-2,4,6-tris-(3,5-di-tertio-butyl-4-hydroxybenzyl)benzène.